# EUROPEAN PATENT SPECIFICATION

(11) **EP 1 283 060 B1**
(45) Date of publication and mention of the grant of the patent: **06.10.2010**
(21) Application number: 01930187.8
(22) Date of filing: 17.05.2001
(51) Int. Cl.: A61K 51/04, A61K 9/30, A61K 47/02, A61K 9/28

(54) **PREPARATIONS FOR DIAGNOSING INFECTION WITH HELICOBACTER PYLORI**
ZUSAMMENSETZUNGEN FÜR DIE DIAGNOSE VON HELICOBACTER PYLORI INFEKTIONEN
PREPARATION SERVANT A DIAGNOSTIQUER LES INFECTIONS PAR L'HELICOBACTER PYLORI

(30) Priority: 19.05.2000 JP 2000148150; 29.08.2000 JP 2000259845
(43) Date of publication of application: 12.02.2003
(73) Proprietor: OTSUKA PHARMACEUTICAL CO., LTD., Chiyoda-ku Tokyo 101-8535 (JP)
(72) Inventor: TSUCHIYA, Kyoko, Boston, MA 02199 (US); OKAMURA, Akio,, Tokyo 143-0011 (JP); KAWASAKI, Junichi, Itano-gun, Tokushima 771-0204 (JP); UNO, Shinichiro,, Tokushima-shi, Tokushima 770-0045 (JP); NODA, Atsunari, Itano-gun, Tokushima 779-0104 (JP); NISHIWAKI, Satoshi, Itano-gun, Tokushima 771-0219 (JP)
(74) Representative: HOFFMANN EITLE
(86) International application number: PCT/JP2001/004122
(87) International publication number: WO 2001/087353

(56) References cited:
- EP-A- 0 787 017
- EP-A- 0 860 170
- EP-A1- 0 878 198
- WO-A-98/53808
- JP-A- 6 157 317

## Description

### FIELD OF THE INVENTION

The present invention relates to a pharmaceutical preparation for use in the diagnosis of Helicobacter pylori infection. More particularly, the invention relates to an oral dosage form which finds application in urea breath testing which is a noninvasive method of detecting Helicobacter pylori.

### DESCRIPTION OF RELATED ART

Since the successful isolation and culture of Helicobacter pylori (hereinafter referred to as H. pylori) by Marshall et al. (Lancet, pp.1273-1275 (1983)), both affirmative and negative views had been advanced on its etiologic role. Recently, however, H. pylori infection has gathered a great deal of attention as a principal cause or a cofactor in the onset of gastritis, peptic ulcer and stomach cancer which are the triad of major upper digestive tract diseases not only in Japan but also abroad. In particular, the recommendation made by NIH Consensus Conference (Bethesda, 1994) that "Peptic ulcer in which an H. pylori infection has been verified, whether it is a primary lesion or a recurrent one, requires eradication therapy supplementing gastric acid antisecretary medication with antibacterials" caused quite a sensation in Japan, urging those concerned to establish an accurate and rapid method for diagnosis of H. pylori infection and verification of eradication of H. pylori.

The technology for detecting H. pylori in the gastric mucosa can be divided into two major categories, namely the invasive one requiring an endoscopy (biopsy) (a bacteriological method involving culture of isolates, a histologic or immunohistologic method for detection, urease test, etc.) and the noninvasive one. Of them, the noninvasive one is preferred from the standpoints of mental and physical burdens on the patient, expediency and safety.

The two representative noninvasive methods currently available are a serologic method for diagnosis which comprises determining the serum level of specific anti-H. pylori antibodies and the urea breath test which comprises administering an isotope carbon-labeled urea orally and determining the labeled carbon dioxide exhaled in breath air (e.g. Sand, J., Gastroenterol, 1996, 31 (suppl) 214, pp.44-46; Gastraenteralogy, 1997, 113, s93-98; Gut, 1994, 35, pp.723-725; Aliment. Pharmacol. Ther. 1997, 11, pp.641-649; Gastraenteralogy, 1995, 109, pp.136-141).

Between the above methods, the serologic method for diagnosis which is based on the presence of specific antibodies has the drawback that since the antibody-positive status of the host's serum persists for at least 3 months even after eradication of H. pylori, approximately 10-15% of the test subjects give false-positive responses and, as such, is not suited for the confirmation of bacterial elimination. Therefore, recently the urea breath test, which is not dependent on the presence of antibodies and is safe and not time-consuming, is broadly employed. This test is based on the property of H. pylori to produce a large amount of the enzyme urease. The urease usually dose not occur in the human body and, therefore, its detection indicates that H. pylori, an urease producer, exists in the stomach. Thus, this method exploits the phenomenon that when the urease-producer H. pylori exists in the host's stomach, the labeled urea ingested by the host decomposes and further reacts with gastric acid so that the labeled urea is converted to the labeled carbon dioxide which is exhaled in breath air (Lancet, pp.174-177 (1987)). A further advantage of this test is that the existence of urease can be tested in a broad region of the stomach.

However, the conventional procedure for this urea breath test involves the intake of an isotope-labeled urea in the form of an aqueous solution and, therefore, various urease-producing bacteria resident in the mouth and throat decompose the ingested urea in an early stage following administration, thus presenting a risk for giving a false-positive test in the diagnosis of H. pylori infection. To prevent this false-positive response, it is necessary to have the test subject gargle his throat with water immediately following ingestion of an aqueous solution of the labeled urea to wash away the labeled urea remaining in the oral cavity or to disinfect the mouth and throat ahead of time.

However, such treatments are burdensome not only to test subjects undergoing the test but also to the physician. Moreover, in order to obtain an accurate result, the detection noise due to the decomposition of urea by the resident bacterial flora in and around the oral cavity must be eliminated by delaying the exhaled air collection time, with the consequent disadvantage of a prolongation of the test procedure.

### SUMMARY OF THE INVENTION

The present invention has for its object to provide an improved oral formulation for a urea breath test. More particularly, the object of the invention is to provide a pharmaceutical preparation with which an H. pylori infection of the gastric mucosa can be detected and diagnosed expediently and noninvasively by the urea breath test and which is free from the risk for a false positive test because of complete elimination of the influence of urease-producing bacteria inhabiting the oral cavity, throat and other tissues excepting the gastrointestinal tract.

A further object of the present invention is to provide a pharmaceutical preparation with which the presence of H. pylori can be detected quickly without a time lag.

To overcome the foregoing disadvantages of the conventional urea breath test, the inventors of the present invention explored in earnest for the development of a pharmaceutical formulation which would show an in vivo behavior such that it remains undissolved in the oral cavity but, upon entry into the stomach, dissolves quickly to allow the labeled urea to disperse rapidly throughout the stomach. As a result, they discovered that a pharmaceutical formulation showing such a favorable in vivo behavior can be provided by using the active substance labeled urea in combination with an excipient component and a lubricant component to prepare a core composition and covering this core composition with a coating agent. Thus, the inventors confirmed that when the above pharmaceutical preparation is administered orally to a test subject, the active substance reaches the stomach without being affected by the urease-producing bacteria resident in the oral cavity and is rapidly dissolved and dispersed in the stomach substantially without being subjected to the retardation of dissolution by the coating and that, therefore, this pharmaceutical preparation is a very useful reagent for the rapid and accurate diagnosis of H. pylori infection. The present invention has been completed on the basis of the above finding.

The present invention, therefore, is concerned with the pharmaceutical preparations defined in the following paragraphs (1)-(14) for the detection of H. pylori infection according to a urea breath test protocol:
(1) A coated preparation for use in the detection of *Helicobacter pylori* infection comprising:
   (i) a core composition comprising:
      19 to 89 parts by weight of isotope carbon-labeled urea relative to 100 parts by weight of the core composition,
      10 to 80 parts by weight of an excipient component relative to 100 parts by weight of the core composition, and
      0.01 to 1 parts by weight of a lubricant component relative to 100 parts by weight of the core composition,
         the excipient component comprising:
         (a) at least one member selected from the group consisting of lactose, sucrose and glucose,
         (b) at least one member selected from the group consisting of crystalline cellulose, low-substitution hydroxypropyl cellulose, carboxymethylcellulose calcium and croscarmellose sodium, and
         (c) at least one member selected from the group consisting of starch, carboxymethylstarch sodium, hydroxypropylstarch, and partially pregelatinized starch; and
   (ii) a coating agent,
      the core composition being coated with 0.1 to 5 parts by weight of the coating agent relative to 100 parts by weight of the core composition.
(2) A coated preparation as def ined in paragraph (1) wherein the proportion of the coating agent is 0.3-5 parts by weight based on 100 parts by weight of the core composition.
(3) A coated preparation as defined in paragraph (1) wherein the prpportion of the coating agent is 0.5-3 parts by weight based on 100 parts by weight of the core composition.
(4) A coated preparation as defined in paragraph (1) containing 25-75 weight % of the isotope carbon-labeled urea, 20-70 weight % of the excipient component and 0.05-0.8 weight % of the lubricant component based on 100 weight % of the core composition.
(5) A coated preparation as defined in paragraph (1) containing 30-70 weight % of the isotope carbon-labeled urea, 35-65 weight % of the excipient component and 0.1-0.7 weight % of the lubricant component based on 100 weight % of the core composition.
(6) A coated composition as defined in paragraph (1) wherein the core composition contains 10-450 parts by weight of the excipient component and 0.01-6 parts by weight of the lubricant component based on 100 parts by weight of the isotope carbon-labeled urea.
(7) A coated preparation as defined in paragraph (1) wherein the core composition contains 50-150 parts by weight of the excipient component and 0.1-5 parts by weight of the lubricant component based on 100 parts by weight of the isotope carbon-labeled urea.
(8) A coated preparation as defined in paragraph (1) wherein the coating agent comprises a water-soluble polymer and a plasticizer.
(9) A coated preparation as defined in paragraph (8) wherein the water-soluble polymer is at least one member selected from the group consisting of pullulan, dextrin, alkali metal alginate, hydroxypropylcellulose, hydroxypropylmethylcellulose, methylcellulose and polyvinylpyrrolidone.
(10) A coated preparation as defined in paragraph (8) wherein the plasticizer is at least one member selected from the group consisting of polyvinyl alcohol, polyethylene glycol, triethyl citrate, triacetin, concentrated glycerin, propylene glycol and polysorbate.
(11) A coated preparation as defined in paragraph (1) wherein, as the excipient component, the core composition contains at least one member selected from the group consisting of lactose, sucrose, glucose, starch, crystalline cellulose, croscarmellose sodium, low-substitution hydroxypropylcellulose, carmellose calcium, crospovidone, carboxymethylstarch sodium, carboxymethylstarch calcium, hydroxypropylstarch, polyvinylpyrrolidone and partly pregelatinized starch.
(12) A coated preparation as defined in paragraph (1) wherein, as the lubricant component, the core composition contains at least one member selected from the group consisting of stearic acid, magnesium stearate, calcium stearate and hydrogenated oil.
(13) A coated preparation as defined in paragraph (1) wherein the core composition contains lactose, crystalline cellulose and starch as the excipient component and magnesium stearate as the lubricant component and the coating agent contains hydroxypropylmethylcellulose, polyethylene glycol, titanium oxide and talc.
(14) A coated preparation as defined in paragraph (1) wherein the isotope carbon-labeled urea is ¹³C-labeled urea.
   The present invention is further concerned with the use of any of the above-defined coated preparations as defined in the following paragraphs (15) and (16):
(15) Use of the coated preparation of paragraph (1) for the manufacture of a urea breath test reagent for detecting a *Helicobacter pylori* infection.
(16) Use of the coated preparation of paragraph (1) for the manufacture of a urea breath test reagent for assessing the *Helicobacter pylori* eradication effect of a *Helicobacter pylori* eradication therapy.

### BRIEF DESCRIPTION OF THE DRAWING

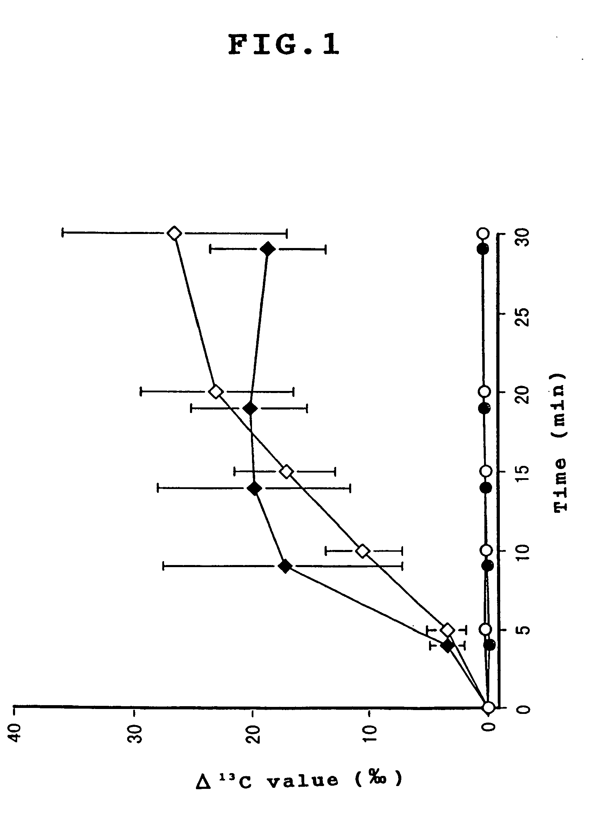

Fig. 1 shows the results of Example 3 in which the ¹³C-labeled urea tablet (coating 2 weight %) was administered orally to test groups (a mouth-washed group and a non-mouth-washed group each consisting of H. pylori-negative and -positive subjects) and the time course of Δ ¹³C value (‰) (the difference in the ¹³CO₂/¹²CO₂ concentration ratio (δ¹³C value) in exhaled air between the exhaled air before and after medication) was monitored. In the diagram, the closed circle represents H. pylori-negative subjects (in mouth-washed group), the open circle represents H. pylori-negative subjects (in the non-mouth-washed group), the closed diamond represents H. pylori-positive subjects (in the mouth-washed group), and the open diamond represents H. pylori-positive subjects (in the non-mouth-washed group). Each graph shows the mean ± standard error for the total test population.

### DETAILED DESCRIPTION OF THE INVENTION

The pharmaceutical preparation according to the present invention is a preparation for use in the detection of H. pylori infection by urea breath testing, **characterized in that** said preparation is a coated preparation comprising a core composition containing an active component and a coating material covering the core composition.

Furthermore, the core composition constituting the coated preparation of the present invention is **characterized in that,** in addition to the active component isotope carbon-labeled urea (hereinafter referred to as the isotope C-labeled urea), the preparation contains an excipient component and a lubricant component each in a herein-defined proportion.

The isotope C-labeled urea for use in the practice of the present invention is urea labeled with an isotope of carbon and serves as an active component for detection of H. pylori infection. As isotopes of carbon, the stable isotope ¹³C and the radioactive isotope ¹¹C or ¹⁴C can be generally mentioned, and as urea labeled by the respective isotopes, ¹³C-labeled urea and ¹¹C-labeled urea or ¹⁴C-labeled urea can be mentioned. These species of labeled urea are invariably used in urea breath tests and all can be used in the present invention as well in the routine manner. Preferably, ¹³C-labeled urea, i.e. urea labeled with the highly stable isotope ¹³C, is used as said isotope C-labeled urea.

The formulating proportion of said isotope C-labeled urea in the core composition is not particularly restricted inasmuch as it is within the range of 19-89 weight % per 100 weight % of the core composition. The preferred proportion is 25-75 weight % and the more preferred proportion is 30-70 weight %.

As the excipient component, the various excipients which are in routine use in the production of pharmaceutical preparations, particularly those in use as excipients for tablets, can be liberally used in the present invention as well. Specifically, there can be mentioned saccharides such as lactose, sucrose, glucose, etc.; water-soluble or water-swellable cellulose derivatives such as crystalline cellulose, low-substitution hydroxypropylcellulose, carboxymethylcellulose calcium(carmellose calcium), croscarmellose sodium, etc.; starch or starch derivatives such as starch, carboxymethylstarch sodium, hydroxypropylstarch, partly pregelatinized starch, etc.; and vinylpyrrolidone derivatives inclusive of polyvinylpyrrolidones such as orospovidone; among others.

These may be used independently or in a suitable combination of two or more species. Preferably, excipients are used in a combination. In this case, preferred is the use of those excipients from at least two groups selected from the following three groups consisting of:
Group 1. saccharides,
Group 2. water-soluble or water-swellable cellulose derivatives,
and Group 3. starch or starch derivatives.
   The combination of these components is not specifically limited. Examples of the combination of the preferable component from the respective groups (lactose from saccharides; crystalline cellulose from water-soluble or water-swellable cellulose derivatives; and starch from starch or starch derivatives) include the combination of lactose and crystalline cellulose or starch; the combination of crystalline cellulose and starch; and the combination of lactose, crystalline cellulose and starch. The preferred excipients are lactose, crystalline cellulose and starch, and it is preferable to use at least two of them in combination. The mode of combination is not particularly restricted but includes the combination of lactose with either crystalline cellulose or starch, the combination of crystalline cellulose with starch, and the combination of lactose with crystalline cellulose and starch.

The formulating proportion of the excipient component in the core composition is not particularly restricted inasmuch as it is within the range of 10-80 weight % but preferably is 20-70 weight %, more preferably 35-65 weight % based on 100 weight % of the core composition. It is also recommended that the formulating proportion of said excipient component based on 100 parts by weight of isotope C-labeled urea should be generally 10-450 parts by weight, preferably 50-150 parts by weight.

With regard to the lubricant component, the various lubricants in routine use in the manufacture of pharmaceutical products, particularly those used as lubricants for tablets, can be liberally employed. Specifically, stearic acid, magnesium stearate, calcium stearate, hydrogenated oil, etc. can be mentioned by way of example. These may be used each independently or in a suitable combination of two or more species. The preferred lubricant is magnesium stearate.

The formulating proportion of said lubricant component in the core composition is not particularly restricted inasmuch as it is within the range of 0.01-1 weight % but is preferably 0.05-0.8 weight %, more preferably 0.1-0.7 weight %, based on 100 weight % of the core composition. Moreover, the preferred proportion of this lubricant component based on 100 parts by weight of isotope C-labeled urea is usually 0.01-6 parts by weight, preferably 0.1-5 parts by weight.

In addition to the above-mentioned components, the core composition for constituting the core of the coated preparation of the present invention may be supplemented with such other components as binder, foaming agent, coloring agent, flavor, corrigent, sweetener, etc. in amounts not interfering with the effect of the invention. As such additional components, the substances which are in routine use in the manufacture of pharmaceutical preparations, particularly tablets, can be liberally employed.

The coated preparation of the present invention is manufactured by using a core comprising at least said isotope C-labeled urea, said excipient component and said lubricant component [uncoated tablet (core tablet), uncoated pill, uncoated granule] and covering its surface with a coating agent.

The coating agent which can be used in the manufacture of the coated preparation of the invention is not particularly restricted but includes a broad variety of coating agents (film-forming agents) in routine use for tablets, pills, granules and so forth. The preferred is a water-soluble coating agent.

The water-soluble coating agent includes polysaccharides which may optionally have a sulfate group, such as pullulan, dextrin and alkali metal salts (e.g. sodium salt, potassium salt) of alginic acid, etc.; water-soluble cellulose derivatives such as cellulose containing 26-33% of methoxy groups, e.g. methylcellulose, and cellulose containing 53.4-77.5% or 7-12% of hydroxypropoxy groups, e.g. hydroxypropylcellulose and hydroxypropylmethylcellulose, etc.; water-soluble polyvinyl derivatives such as polyvinylpyrrolidone, polyvinyl alcohol, etc.; enteric polymers such as carboxymethylethylcellulose, hydroxypropylmethylcellulose phthalate, cellulose acetate phthalate, hydroxypropylmethylcellulose acetate succinate, methacrylic copolymer, carboxymethylethylcellulose, etc.; gastric polymers such as polyvinyl acetal diethylaminoacetate, aminoalkyl methaorylate copolymer E, etc.; and sustained-release polymers such as ethylcellulose etc. These may be used each alone or in a combination of two or more species.

The preferred, among these water-soluble polymers, are pullulan, dextrin, alkali metal alginate (sodium alginate, potassium alginate), hydroxypropylcellulose, hydroxypropylmethylcellulose, methylcellulose and polyvinylpyrrolidone, and the still more preferred are hydroxypropylcellulose and hydroxypropylmethylcellulose.

The coating agent for use in the present invention may be one containing only a single species of said water-soluble polymer or one containing two or more species in a suitable combination.

In the present invention, a coating agent comprising such a water-soluble polymer is preferably used in conjugation with a plasticizer. As such plasticizer, the plasticizers in routine use in coating compositions can be selectively used and specifically polyhydric alcohols such as polyethylene glycols inclusive of macrogol 6000, macrogol 4000, etc., concentrated glycerin, propylene glycol, polyvinyl alcohol, etc.; triethyl citrate; triacetin; and surfactants such as polysorbate (e.g. Tween 80) can be mentioned as examples.

These plasticizers can each be used in conjunction with said water-soluble polymer or be used in a combination of two or more in conjunction with the water-soluble polymer. As the preferred plasticizer, polyvinyl alcohol, polyethylene glycol, triethyl citrate, triacetin, concentrated glycerin, propylene glycol or polysorbate can be mentioned. Among them, polyethylene glycol is preferred and macrogol 6000 is the more preferred.

The mode of combination of said water-soluble polymer with said plasticizer is not particularly restricted but includes, to mention a few preferred examples, the combination of hydroxypropylcellulose with polyethylene glycol, triethyl citrate or triaoetin and the combination of hydroxypropylmethyloellulose with polyethylene glycol, triethyl citrate or triacetin. The more preferred combination is the combination of hydroxypropylmethyloellulose with polyethylene glycol.

The coating agent for use in the present invention may be supplemented with a coloring agent such as a pigment or a dye, a flavor, a oorrigent and/or a sweetener each in an amount not interfering with the effect of the present invention. As such formulating additives, those in routine use in pharmaceutical formulations, particularly in coating formulations, can be liberally employed. As examples of the coloring agent, titanium oxide, talc, iron oxide red, etc. can be mentioned. The preferred are titanium oxide and talc. The coloring agent is intended to impart a desired color to the coated preparation of the invention and its amount is not particularly restricted inasmuch as it is sufficient to satisfy the need. Usually, such a coloring agent is used in a proportion of 1-70 weight %, preferably 5-50 weight %, based on 100 weight % of the coating agent. When talc and titanium oxide are used in combination, their ratio in the coating agent may be 25-175 weight parts, preferably 50-150 weight parts, of talc to 100 weight parts of titanium oxide.

The proportion of the coating agent to be used for covering the core composition [e.g. uncoated tablet (core tablet), uncoated pill, uncoated granule] can be judiciously selected from the range of 0.1-10 parts by weight based on 100 parts by weight of the core composition and is preferably 0.3-5 parts by weight, more preferably 0.5-3 parts by weight. If the proportion exceeds 10 parts by weight in a large measure, the dissolution of the coating film in the stomach is retarded to cause a marked delay in the dispersion and dissolution of the core composition, with the consequent disadvantage of frustrating a rapid diagnosis. On the other hand, if the proportion is by far smaller than 0.1 parts by weight, the influence of urease-producing bacteria in the mouth and throat may not be excluded so that a false-positive result tends to result.

The method of covering the core composition with said coating agent is not particularly restricted but the coating can be performed in the usual manner according to the form of the core composition or final preparation.

The form of the coated preparation of the invention is not particularly restricted inasmuch as the operation and result of the invention may be implemented, thus including tablets, pills and granules, among others. The preferred are tablets and pills, with tablets being particularly preferred. These forms of the coated preparation can be manufactured by the methods established in the art.

Taking the manufacture of tablets as an example, the coated preparation of the invention can be manufactured by preparing a core composition containing said at least 3 components (isotope C-labeled urea, excipient component and lubricant component) in tablet form and covering the surface of the core tablet with said coating agent. The core tablets can be produced by the granulation compression method (indirect compression method) which comprises blending the two components other than the lubricant component (namely, isotope C-labeled urea and excipient component), optionally together with other suitable additive components, granulating the mixture, adding the lubricant component, and compressing the whole mixture; or by the direct powder compression method (direct compression method) which comprises blending said 3 components, optionally together with other suitable additives, uniformly and directly compressing the whole mixture. Although whichever of said direct powder compression method and said granulation compression method can be used as mentioned above, the direct powder compression method is preferred because the granulating step can be dispensed with. The covering with the coating agent can be carried out in the conventional manner, for example by the method using a coating pan or the method using a fluid-bed coating equipment.

The coated preparation of the present invention is prepared in such a manner that it contains 10-300 mg, preferably 50-150 mg, of the active component isotope C-labeled urea per dosage unit.

The coated preparation of the present invention is useful for the detection of H. pylori infection.

Further, the present invention provides using the above-described coated preparation of the invention for the manufacture of a urea breath test reagent in the conventional urea breath test for the detection of H. pylori infection. Specifically, after the coated preparation of the invention is orally administered to a test subject and the subject's exhaled air is collected, the H. pylori infection is detected based on the ratio of isotope carbon-labeled CO₂ contained in the exhaled air measured as a ratio of isotope carbon-labeled CO₂ to ¹²CO₂.

In this case, the timing of collecting the air exhaled after the administration of the coated preparation is not particularly limited. For example, after the administration of the coated preparation of the invention, the ratio of isotope carbon-labeled CO₂ to ¹²CO₂ can be repeatedly measured by collecting exhaled air over time. However, as understood from the results of Example 3 (Fig. 1) described below, H. pylori positive and H. pylori negative subjects are clearly different in Δ isotope carbon-labeled C value (‰) after the administration of the coated preparation of the invention (e.g., the difference in the ¹³CO₂/¹²CO₂ concentration ratio ( δ ¹³C value) of the exhaled air at each sampling time after administration and of the exhaled air before tablet administration). Therefore, the H. pylori infection can be detected by measuring the Δ isotope carbon-labeled C value (‰) at any sampling time after the administration. Specifically, exhaled air is usually collected, for example, 5 to 60 minutes, preferably 10 to 30 minutes, after the administration.

For the measurement and analysis of the labeled CO₂ contained in an exhaled air sample, conventional analysis methods can be used such as liquid scintillation counter method, mass spectrometry, infrared spectroscopy, emission spectrometry, magnetic resonance spectrum and the like. Preferred are infrared spectrometry and mass spectrometry because of their measurement accuracy.

Usually, the coated preparation of the invention is preferably administered orally with water in the fasted state. The amount of the labeled urea contained in a dosage form of the preparation of the invention is not particularly limited. It is usually selected and adjusted from a range of 10 to 300 mg per dose unit.

Also, the coated preparation of the present invention is useful for assessment of the bacterial elimination effect after eradication therapy. This assessment is conducted by administering the coated preparation of the invention to a patient having had the H. pylori eradication therapy and measuring the ratio of isotope carbon-labeled CO₂ contained in the patient's exhaled air obtained as a ratio of isotope carbon-labeled CO₂ to ¹²CO₂ according to the urea breath test. The procedure for detection or assessment is not particularly restricted but a typical protocol may comprise causing a patient having had the H. pylori eradication therapy to administrer the coated preparation, e.g. a preparation containing 10-300 mg of ¹³C-labeled urea per dose unit, orally together with water in the fasted state, collecting exhaled air directly in a exhaled air bag after 5-60 minutes, preferably 10-20 minutes, and analyzing the same in a mass spectrometer for measurement of the ¹³CO₂/¹²CO₂ ratio in the exhaled air.

### EXAMPLES

The following reference and working examples illustrate the present invention in further detail.

### Reference Example 1

The components indicated in Table 1 were blended in the indicated proportions and compressed by the direct compression method to prepare tablets of Reference Example 1. These tablets were tested as directed in Japanese Pharmacopeia (XIII), Dissolution Test [Method 2 (paddle method)] using water as the test solution at a bath temperature of 37±0.5°C and a paddle speed of 50 rpm. The ratio of dissolved urea (%) after 20 and 60 seconds were determined. The results are shown also in Table 1.

**Table 1**

| Core composition | Formulating amount |
|---|---|
| Urea | 100.0 mg |
| Lactose | 34.4 mg |
| Crystalline cellulose | 60.0 mg |
| Corn starch | 5.0 mg |
| Magnesium stearate | 0.6 mg |
| Ratio of dissolved urea (after 20 sec), average | 3.1% |
| Ratio of dissolved urea (after 60 sec), average | 46.6% |

The above results suggested that the dissolution of tablets of the formulation containing at least urea, an excipient component (lactose, crystalline cellulose, corn starch) and a lubricant (magnesium stearate) as tablet components is so rapid that when administered by the oral route, they dissolve quickly in the oral cavity.

### Example 1

Tablets were manufactured according to the same formulation as used in Reference Example 1 except that an isotope (¹³C)-labeled urea was used in lieu of urea. These core tablets were coated with an aqueous composition of hydroxypropylmethylcellulose/polyethylene glycol/titanium oxide/talc (6/3/1/1, by weight) in a coating proportion of 2 parts by weight based on 100 parts by weight of the core tablet by the coating method which is used generally in tablet manufacture to prepare coated tablets of the present invention. As in Reference Example 1, these coated tablets were tested in accordance with Japanese Pharmacopeia (XIII), Dissolution Test, Method 2 (paddle method), using water as the test solution at a bath temperature of 37±0.5°C and a paddle speed of 50 rpm, and the ratio of dissolved ¹³C-urea (%) after 20 and 60 seconds and 10 minutes were determined. The formulations used and dissolution test results are shown in Table 2.

**Table 2**

| Formulation | | Formulating amount |
|---|---|---|
| Core | ¹³C-urea | 100.0 mg |
| | Lactose | 34.4 mg |
| | Crystalline cellulose | 60.0 mg |
| | Corn starch | 5.0 mg |
| | Magnesium stearate | 0.6 mg |
| Coating | Hydroxypropylmethylcellulose | 2.4 mg |
| | Polyethylene glycol | 0.8 mg |
| | Titanium oxide | 0.4 mg |
| | Talc | 0.4 mg |
| Ratio of dissolved urea (after 20 sec), average | | 0.0% |
| Ratio of dissolved urea (after 60 sec), average | | 11.8% |
| Ratio of dissolved urea (after 10 min), average | | 93.1% |

The above results indicate that the release of urea was not observed at 20 sec. following administration and was only slight at even 60 sec. It was, therefore, clear that as the coated tablet of the invention is swallowed together with water in the usual manner, the tablet finds its way into the stomach without dissolving in the oral cavity and releases the isotope-labeled urea in the stomach and that, therefore, a gastric H. pylori infection can be detected without being confounded by the urease present in the oral cavity.

### Example 2

The proper proportion of the coating agent relative to the core tablet by weight was explored based on the result of Example 1. Thus, coating solutions were prepared in coating-core weight ratios over the range of 0 to 20 parts by weight (0, 0.1, 0.3, 0.5, 1, 2, 3, 5, 10, 15 and 20 parts by weight) relative to 100 parts by weight of the core tablet in the same manner as in Example 1 and the disintegration test was performed on each coated tablet to measure its lag time preceding disintegration and disintegration time. The core composition of the coated tablets used in the test are shown below.

### <Core>

| | |
|---|---|
| ¹³C-urea | 100.0 mg |
| Lactose (Dilactose S, Freund Ind.) | 34.4 mg |
| Crystalline cellulose (Avicel PH-101, Asahi Kasei) | 60.0 mg |
| Corn starch | 5.0 mg |
| Magnesium stearate | 0.6 mg |

Coated tablets containing 0.1-20 parts by weight of the coating agent based on 100 parts by weight of the above core were prepared by using coating solutions containing the following components in a coating proportion of 1 or 8 weight %.

### <Coating solution>

| | |
|---|---|
| Hydroxypropylcellulose (TC-5RW, Shin-Etsu Chemical) | 6 wt. parts |
| Macrogol 6000 | 2 wt. parts |
| Titanium oxide | 1 wt. parts |
| Talc | 1 wt. parts |

### (1) Measurement of disintegration time (disintegration test)

Each coated tablet prepared as above was tested as directed in Japanese Pharmacopoeia (XIII), Disintegration Test ["Tablets coated with suitable coating agents"] using water as the test solution and a bath temperature of 37±2° C, (6 test tablets). The time which elapsed until no residues of the test tablet were detected in the glass tube any longer or, if any residue was present, it was a filmy or spongy substance or only a soft or sludge-like substance was slightly detected was measured and recorded as disintegration time.

### (2) Measurement of lag time (dissolution test)

Each coated tablet prepared above was tested as directed in Japanese Pharmacopoeia (XIII), Dissolution Test [Method 2 (paddle method)] using water (500 ml) as the test solution at a bath temperature of 37±0.5° C and a paddle speed of 75 rpm, and the time from the start of paddle rotation to the start of tablet disintegration was measured and regarded as lag time.

The disintegration time and lag time data generated by the above tests with the coated tablets are shown in Table 3.

**Table 3**

| | | | | | |
|---|---|---|---|---|---|
| Coating proportion (parts by weight) | 0 | 0.1 | 0.3 | 0.5 | 2.0 |
| Disintegration time | 10"-15" | 10"-15" | 10"-15" | 10"-15" | 10"-15" |
| Lag time | - | 5 sec. | 10-15 15-20 sec. . | 20-40 sec. | 20-40 sec. |
| | | | | | |
| Coasting proportion (parts by weight) | 3.0 | 5.0 | 10.0 | 15.0 | 20.0 |
| Disintegration time | 15"-20" | 45"-55" | 1'55"- 2'35" | 2'20"- 3'10" | 3'30"- 4'10" |
| Lag time | 20-40 sec. | 20-40 sec. | 1-2 min. | 2-3 min. | 3-4 min. |

The disintegration test revealed that while the coated preparations corresponding to the coating proportions of 0-3 parts by weight showed little variation in disintegration time, the coated preparation corresponding to the coating proportion of 5 parts by weight showed slight retardation and, when the coating proportion exceeded 10 parts by weight, it took 2 minutes or longer for the coated preparations to disintegrate. The dissolution test revealed that the start of release of the core composition could be retarded (induction of a lag time) by 0.1 parts by weight of coating and that this lag time could be prolonged by increasing the coating proportion to 0.3 parts by weight or more, preferably not less than 0.5 parts by weight. There was little difference in the lag time among the coated preparations over the coating proportion range of 1-5 parts by weight. However, as the coating proportion exceeded 10 parts by weight, the lag time was considerably prolonged, suggesting that the actual release of the core composition would be delayed. These findings suggested that the preferred proportion of the coating agent based on 100 parts by weight of the core composition is generally 0.1-10 parts by weight, more preferably 0.3-5 parts by weight, still more preferably 0.5-3 parts by weight, particularly 1-3 parts by weight.

### Example 3

Using the coated preparation obtained in Example 1, the following experiment was performed.

Thus, by carrying out the ¹³C-labeled urea breath test using a solution of ¹³C-urea in adult men, 14 H. pylori-negative subjects and 6 H. pylori-positive subjects, or a total of 20 subjects, were selected, and the following experiment was performed in these subjects.

First, the above 20 subjects were divided into two groups, Group A and Group B (each group: 7 H. pylori-negative and 3 H. pylori-positive subjects) and the ¹³C-labeled urea breath test using the coated preparation of the invention was performed twice, 7 days apart, in each subject. Thus, in Group A, mouth washing was carried out at the first breath test (mouth-washed) but not carried out at the second breath test (non-mouth-washed). In Group B, mouth washing was not carried out at the first breath test (non-mouth-washed) but carried out at the second breath test (mouth-washed).

The breath test was performed as follows. Each subject was instructed to ingest the coated preparation together with 100 mL of water and the exhaled air was collected into an aluminum-laminated bag of about 300 mL capacity at 6 points of time, namely before tablet intake and 5 min, 10 min, 15 min, 20 min, and 30 min after intake. The exhaled air thus collected was analyzed using an automatic ¹³CO₂ urea breath analyzer (GC-MS, tradename: ABCA-G (Europe Scientific)). Thus, for each exhaled air sample transferred from the aluminum-laminated bag to the exclusive reduced-pressure sampling tube, the δ ¹³C value (%) (the ¹³CO₂/¹²CO₂ concentration ratio of the exhaled air at each sampling time) was determined. Then, the Δ ¹³C value (‰), which is the difference between the δ ¹³C value (‰) of the exhaled air sample before tablet intake and the δ ¹³C value (‰) of the exhaled air at each sampling time after intake, was calculated.

The Δ¹³C value (‰) [the difference in the ¹³CO₂/¹²CO₂ concentration ratio (δ¹³C value) of the exhaled air before tablet administration and the exhaled air at each sampling time after administration] was determined after oral administration in the respective test groups (the mouth-washed group and non-mouth-washed group each consisting of H. pylori-negative and H. pylori-positive subjects) and the results are shown in Fig. 1. In Fig. 1, the closed circle represents the result for 14 H. pylori-negative subjects (mouth-washed group), the open circle represents the result for 14 H. pylori-negative subjects (non-mouth-washed group), the closed diamond represents the result for 6 H. pylori-positive subjects (mouth-washed group), and the open diamond represents the result for 6 H. pylori-positive subjects (non-mouth-washed group). Each graph shows the mean ± standard error for the total test population.

### (1) Influence of bacteria resident in the mouth and throat

It is apparent from Fig. 1 showing the test results in H. pylori-negative subjects (indicated by the closed circle and open circle on the drawing) that when the coated preparation of the present invention was used as a test reagent, omission of mouth washing did not introduce a change in Δ ¹³C value that might be attributed to the influence of mouth and throat bacteria.

### (2) The time-course pattern of Δ¹³C value (‰) in H. pylori-positive patients

Whereas the Δ ¹³C value (‰) reflecting the urease activity of H. pylori in the stomach could be detected in H. pylori-positive subjects (indicated by the closed and open diamonds on the drawing), little change was found in the Δ ¹³C value (‰) in H. pylori-negative subjects (indicated by the closed and open circles on the drawing) as mentioned above. It was, therefore, evident that by using the coated preparation of the present invention as a diagnostic reagent, the Δ ¹³C value (‰) in a H. pylori-positive subject and the Δ ¹³C value (‰) in a H. pylori-negative subject can be detected with a clear distinction, hence a H. pylori-positive patient and a H. pylori-negative patient can be accurately sorted out, that is to say a H. pylori infection can be accurately diagnosed. It was confirmed from the above results that by covering the uncoated tablet (core tablet) containing ¹³C-labeled urea and other components with the coating agent at the defined coating rate, the influence of the urease-producing bacteria in the mouth and throat can be completely excluded, thus permitting an accurate diagnosis of a H. pylori infection.

### INDUSTRIAL APPLICABILITY

The conventional diagnostic reagent for H. pylori infection gives false-positive results at times, for because the reagent powder of isotope C-labeled urea is dissolved in water and administered in the form of an aqueous solution, the test result tends to be confounded by the urease-producing bacteria in the mouth and throat. Therefore, in order that an accurate determination may be made, it is necessary to have the mouth washed immediately after administration of the labeled urea-containing solution or perform a determination on the exhaled air collected at least 20 minutes after administration by which time the influence of the resident bacteria in the oral cavity may have diminished, among other restrictions.

With the coated preparation of the present invention, the influence of the urease-producing bacteria resident in the mouth and throat is completely excluded so that the test is not subject to the above restrictions. Moreover, since the dissolution and dispersion of the active component in the stomach are rapid, it is possible to collect exhaled air early after administration and measure the labeled carbon dioxide to make a rapid determination of H. pylori infection. Furthermore, exclusion of the influence of oral bacteria means that the out-off value as a criterion of H. pylori infection can be set more stringent and low to thereby further improve the detection accuracy and reduce the time required for determination. Therefore, the coated preparation of the present invention is considered to be a very useful preparation for use as a diagnostic reagent for H. pylori infection yielding more rapid, expedient and accurate test results.

## Claims

1. A coated preparation for use in the detection of *Helicobacter pylori* infection comprising:
(i) a core composition comprising:
19 to 89 parts by weight of isotope carbon-labeled urea relative to 100 parts by weight of the core composition,
10 to 80 parts by weight of an excipient component relative to 100 parts by weight of the core composition, and
0.01 to 1 parts by weight of a lubricant component relative to 100 parts by weight of the core composition,
the excipient component comprising:
(a) at least one member selected from the group consisting of lactose, sucrose and glucose,
(b) at least one member selected from the group consisting of crystalline cellulose, low-substitution hydroxypropyl cellulose, carboxymethylcellulose calcium and croscarmellose sodium, and
(c) at least one member selected from the group consisting of starch, carboxymethylstarch sodium, hydroxypropylstarch, and partially pregelatinized starch; and
(ii) a coating agent,
the core composition being coated with 0.1 to 5 parts by weight of the coating agent relative to 100 parts by weight of the core composition.

2. A coated preparation according to Claim 1 whereon the proportions of the coating agent is 0.3-5 parts by weight based on 100 parts by weight of the core composition.

3. A coated preparation according to Claim 1 wherein the proportions of the coating agent is 0.5-3 parts by weight based on 100 parts by weight of the core composition.

4. A coated preparation according to Claim 1 containing 25-75 weight % of the isotope carbon-labeled urea, 20-70 weight % of the excipient component and 0.05-0.8 weight % of the lubricant component based on 100 weight % of the core composition.

5. A coated preparation according to Claim 1 containing 30-70 weight % of the isotope carbon-labeled urea, 35-65 weight % of the excipient component and 0.1-0.7 weight % of the lubricant component based on 100 weight % of the core composition.

6. A coated composition according to Claim 1 wherein the core composition contains 10-450 parts by weight of the excipient component and 0.01-6 parts by weight of the lubricant component based on 100 parts by weight of the isotope carbon-labeled urea.

7. A coated preparation according to Claim 1 wherein the core composition contains 50-150 parts by weight of the excipient component and 0.1-5 parts by weight of the lubricant component based on 100 parts by weight of the isotope carbon-labeled urea.

8. A coated preparation as claimed in Claim 1 wherein the coating agent comprises a water-soluble polymer and a plasticizer.

9. A coated preparation according to Claim 8 wherein the water-soluble polymer is at least one member selected from the group consisting of pullulan, dextrin, alkali metal alginate, hydroxypropylcellulose. hydroxypropylmethylcellulose, methylcellulose and polyvinylpyrrolidone.

10. A coated preparation according to Claim 8 wherein the plasticizer is at least one member selected from the group consisting of polyvinyl alcohol, polyethylene glycol, triethyl citrate, triacetin, concentrated glycerin, propylene glycol and polysorbate.

11. A coated preparation according to claim 1 wherein, as excipient component, the core composition further contains at least one member selected from the group consisting of crospovidone, carboxymethylstarch calcium and polyvinylpyrrolidone

12. A coated preparation according to Claim 1 wherein, as the lubricant component, the core composition contains at least one member selected from the group consisting of stearic acid, magnesium stearate, calcium stearate and hydrogenated oil.

13. A coated preparation according to Claim 1 wherein the core composition contains lactose, crystalline cellulose and starch as the excipient component and magnesium stearate as the lubricant component and the coating agent contains hydroxypropylmethylcellulose, polyethylene glycol, titanium oxide and talc.

14. A coated preparation according to Claim 1 wherein the isotope carbon-labeled urea is ¹³C-labeled urea.

15. Use of the coated preparation of Claim 1 for the manufacture of a urea breath test reagent for detecting a *Helicobacter pylori* infection.

16. Use of the coated preparation of Claim 1 for the manufacture of a urea breath test reagent for assessing the *Helicobacter pylori* eradication effect of a *Helicobacter pylori* eradication therapy.

## Patentansprüche

1. Eine überzogene Zubereitung zur Verwendung bei der Detektion einer Helicobacter pylori-Infektion, umfassend:
(i) eine Kernzusammensetzung, umfassend:
19 bis 89 Gew.-Teile von Kohlenstoffisotopmarkiertem Harnstoff, bezogen auf 100 Gew.-Teile der Kernzusammensetzung,
10 bis 80 Gew.-Teile eines Hilfsstoffbestandteils, bezogen auf 100 Gew.-Teile der Kernzusammensetzung, und
0,01 bis 1 Gew.-Teil eines Schmiermittelbestandteils, bezogen auf 100 Gew.-Teile der Kernzusammensetzung,
wobei der Hilfsstoffbestandteil umfasst:
(a) mindestens ein Element, ausgewählt aus der Gruppe bestehend aus Lactose, Saccharose und Glucose,
(b) mindestens ein Element, ausgewählt aus der Gruppe bestehend aus kristalliner Cellulose, niedrig-substituierter Hydroxypropylcellulose, Carboxymethylcellulose-Calcium und Croscarmellose-Natrium, und
(c) mindestens ein Element, ausgewählt aus der Gruppe bestehend aus Stärke, Carboxymethylstärke-Natrium, Hydroxypropylstärke und teilweise prägelatinierter Stärke; und
(ii) ein Überzugsmittel,
wobei die Kernzusammensetzung mit 0,1 bis 5 Gew.-Teilen des Überzugsmittels, bezogen auf 100 Gew.-Teile der Kernzusammensetzung, überzogen ist.

2. Überzogene Zubereitung gemäß Anspruch 1, wobei der Anteil des Überzugsmittels 0,3 bis 5 Gew.-Teile, bezogen auf 100 Gew.-Teile der Kernzusammensetzung, beträgt.

3. Überzogene Zubereitung gemäß Anspruch 1, wobei der Anteil des Überzugsmittels 0,5 bis 3 Gew.-Teile, bezogen auf 100 Gew.-Teile der Kernzusammensetzung, beträgt.

4. Überzogene Zubereitung gemäß Anspruch 1, enthaltend 25 bis 75 Gew.% des Kohlenstoffisotop-markierten Harnstoffs, 20 bis 70 Gew.% des Hilfsstoffbestandteils und 0,05 bis 0,8 Gew.% des Schmiermittelbestandteils, bezogen auf 100 Gew.% der Kernzusammensetzung.

5. Überzogene Zubereitung gemäß Anspruch 1, enthaltend 30 bis 70 Gew.% des Kohlenstoffisotop-markierten Harnstoffs, 35 bis 65 Gew.% des Hilfsstoffbestandteils und 0,1 bis 0,7 Gew.% des Schmiermittelbestandteils, bezogen auf 100 Gew.% der Kernzusammensetzung.

6. Überzogene Zubereitung gemäß Anspruch 1, wobei die Kernzusammensetzung 10 bis 450 Gew.-Teile des Hilfsstoffbestandteils und 0,01 bis 6 Gew.-Teile des Schmiermittelbestandteils, bezogen auf 100 Gew.-Teile des Kohlenstoffisotop-markierten Harnstoffs, enthält.

7. Überzogene Zubereitung gemäß Anspruch 1, wobei die Kernzusammensetzung 50 bis 150 Gew.-Teile des Hilfsstoffbestandteils und 0,1 bis 5 Gew.-Teile des Schmiermittelbestandteils, bezogen auf 100 Gew.-Teile des Kohlenstoffisotop-markierten Harnstoffs, enthält.

8. Überzogene Zubereitung gemäß Anspruch 1, wobei das Überzugsmittel ein wasserlösliches Polymer und einen Weichmacher umfasst.

9. Überzogene Zubereitung gemäß Anspruch 8, wobei das wasserlösliche Polymer mindestens ein Element, ausgewählt aus der Gruppe bestehend aus Pullulan, Dextrin, Alkalimetallalginat, Hydroxypropylcellulose, Hydroxypropylmethylcelluose, Methylcellulose und Polyvinylpyrrolidon, ist.

10. Überzogene Zubereitung gemäß Anspruch 8, wobei der Weichmacher mindestens ein Element, ausgewählt aus der Gruppe bestehend aus Polyvinylalkohol, Polyethylenglycol, Triethylcitrat, Triacetin, konzentriertem Glycerin, Propylenglycol und Polysorbat, ist.

11. Überzogene Zubereitung gemäß Anspruch 1, wobei die Kernzusammensetzung als Hilfsstoffbestandteil ferner mindestens ein Element, ausgewählt aus der Gruppe bestehend aus Crospovidon, Carboxymethylstärke-Calcium und Polyvinylpyrrolidon, enthält.

12. Überzogene Zubereitung gemäß Anspruch 1, wobei die Kernzusammensetzung als Schmiermittelbestandteil mindestens ein Element, ausgewählt aus der Gruppe bestehend aus Stearinsäure, Magnesiumstearat, Calciumstearat und einem hydrierten Öl, enthält.

13. Überzogene Zubereitung gemäß Anspruch 1, wobei die Kernzusammensetzung Lactose, kristalline Cellulose und Stärke als Hilfsstoffbestandteil und Magnesiumstearat als Schmiermittelbestandteil enthält und das Überzugsmittel Hydroxypropylmethylcellulose, Polyethylenglycol, Titanoxid und Talkum enthält.

14. Überzogene Zubereitung gemäß Anspruch 1, wobei der Kohlenstoffisotop-markierte Harnstoff ¹³C-markierter Harnstoff ist.

15. Verwendung der überzogenen Zubereitung gemäß Anspruch 1 für die Herstellung eines Harnstoff-Atemtest-Reagenzes für die Erkennung einer Helicobacter pylori-Infektion.

16. Verwendung der überzogenen Zubereitung gemäß Anspruch 1 für die Herstellung eines Harnstoff-Atemtest-Reagenzes für die Beurteilung des Helicobacter pylori-Erradikationseffekts einer Helicobacter pylori-Erradikationstherapie.

## Revendications

1. Préparation enrobée pour une utilisation dans la détection d'une infection à *Helicobacter pylori* comprenant :
(i) une composition centrale comprenant :
19 à 89 parties en poids d'urée marquée par un isotope du carbone par rapport à 100 parties en poids de la composition centrale,
10 à 80 parties en poids d'un composant excipient par rapport à 100 parties en poids de la composition centrale, et
0,01 à 1 partie en poids d'un composant lubrifiant par rapport à 100 parties en poids de la composition centrale,
le composant excipient comprenant :
(a) au moins un membre choisi dans le groupe constitué de lactose, de saccharose et de glucose,
(b) au moins un membre choisi dans le groupe constitué de cellulose cristalline, d'hydroxypropylcellulose faiblement substituée, de carboxyméthylcellulose calcique et de croscarmellose sodique, et
(c) au moins un membre choisi dans le groupe constitué d'amidon, de carboxyméthylamidon sodique, d'hydroxypropylamidon et d'amidon partiellement prégélatinisé ; et
(ii) un agent d'enrobage,
la composition centrale étant enrobée avec 0,1 à 5 parties en poids de l'agent d'enrobage par rapport à 100 parties en poids de la composition centrale.

2. Préparation enrobée selon la revendication 1, dans laquelle la proportion de l'agent d'enrobage est de 0,3 à 5 parties en poids par rapport à 100 parties en poids de la composition centrale.

3. Préparation enrobée selon la revendication 1, dans laquelle la proportion de l'agent d'enrobage est de 0,5 à 3 parties en poids par rapport à 100 parties en poids de la composition centrale.

4. Préparation enrobée selon la revendication 1, contenant 25 à 75 % en poids d'urée marquée par un isotope du carbone, 20 à 70 % en poids du composant excipient et 0,05 à 0,8 % en poids du composant lubrifiant par rapport à 100 % en poids de la composition centrale.

5. Préparation enrobée selon la revendication 1, contenant 30 à 70 % en poids d'urée marquée par un isotope du carbone, 35 à 65 % en poids du composant excipient et 0,1 à 0,7 % en poids du composant lubrifiant par rapport à 100 % en poids de la composition centrale.

6. Préparation enrobée selon la revendication 1, dans laquelle la composition centrale contient 10 à 450 parties en poids du composant excipient et 0,01 à 6 parties en poids du composant lubrifiant par rapport à 100 parties en poids d'urée marquée par un isotope.

7. Préparation enrobée selon la revendication 1, dans laquelle la composition centrale contient 50 à 150 parties en poids du composant excipient et 0,01 à 5 parties en poids du composant lubrifiant par rapport à 100 parties en poids d'urée marquée par un isotope.

8. Préparation enrobée selon la revendication 1, dans laquelle l'agent d'enrobage comprend un polymère hydrosoluble et un plastifiant.

9. Préparation enrobée selon la revendication 8, dans laquelle le polymère hydrosoluble est au moins un membre choisi dans le groupe constitué de pullulane, de dextrine, d'un alginate de métal alcalin, d'hydroxypropylcellulose, d'hydroxypropylméthylcellulose, de méthyl-cellulose et de polyvinylpyrrolidone.

10. Préparation enrobée selon la revendication 8, dans laquelle le plastifiant est au moins un membre choisi dans le groupe constitué de polyalcool vinylique, de polyéthylène glycol, de citrate de triéthyle, de triacétine, de glycérine concentrée, de propylène glycol et de polysorbate.

11. Préparation enrobée selon la revendication 1, dans laquelle, en tant que composant excipient, la composition centrale contient en outre au moins un membre choisi dans le groupe constitué de crospovidone, de carboxyméthyl-amidon calcique et de polyvinylpyrrolidone.

12. Préparation enrobée selon la revendication 1, dans laquelle, en tant que composant lubrifiant, la composition centrale contient au moins un membre choisi dans le groupe constitué d'acide stéarique, de stéarate de magnésium, de stéarate de calcium et d'huile hydrogénée.

13. Préparation enrobée selon la revendication 1, dans laquelle la composition centrale contient du lactose, de la cellulose cristalline et de l'amidon en tant que composant excipient et du stéarate de magnésium en tant que composant lubrifiant et l'agent d'enrobage contient de l'hydroxypropylméthylcellulose, du polyéthylène glycol, de l'oxyde de titane et du talc.

14. Préparation enrobée selon la revendication 1, dans laquelle l'urée marquée par un isotope du carbone est de l'urée marquée au ¹³C.

15. Utilisation de la préparation enrobée selon la revendication 1, pour la fabrication d'un réactif de test respiratoire à l'urée pour la détection d'une infection à *Helicobacter pylori.*

16. Utilisation de la préparation enrobée selon la revendication 1, pour la fabrication d'un réactif de test respiratoire à l'urée pour estimer l'effet d'éradication de *Helicobacter pylori* d'une thérapie d'éradication de *Helicobacter pylori.*
